# EUROPEAN PATENT SPECIFICATION

(11) **EP 1 803 395 B1**
(45) Date of publication and mention of the grant of the patent: **26.01.2011**
(21) Application number: 06256631.0
(22) Date of filing: 29.12.2006
(51) Int. Cl.: A61B 5/06

(54) **Magnetic stabilization of catheter location sensor**
Magnetische Stabilisierung eines Kartierungskatheters
Stabilisation magnétique d'un cathéter de cartographie

(30) Priority: 30.12.2005 US 322594
(43) Date of publication of application: 04.07.2007
(73) Proprietor: Biosense Webster, Inc., Diamond Bar, CA 91765 (US)
(72) Inventor: Grunewald, Debby Esther, Los Angeles, CA 90007 (US); Schmidt, Jerry Author, Coto de Caza, CA 92679 (US); Pendekanti, Rajesh, Chino Hills, CA 91709 (US)
(74) Representative: Belcher, Simon James

(56) References cited:
- WO-A-01/21069

## Description

This invention is directed to medical catheters, in particular, catheters that carry location sensors for cardiac mapping and ablation.

Electrode catheters have been in common use in medical practice for many years. They are used to stimulate and map electrical activity in the heart and to ablate sites of aberrant electrical activity.

In use, the electrode catheter is inserted into a major vein or artery, e.g., femoral artery, and then guided into the chamber of the heart which is of concern. Once the catheter is positioned within the heart, the location of aberrant electrical activity within the heart is then located.

One location technique involves an electrophysiological mapping procedure whereby the electrical signals emanating from the conductive endocardial tissues are systematically monitored and a map is created of those signals. By analyzing that map, the physician can identify the interfering electrical pathway. A conventional method for mapping the electrical signals from conductive heart tissue is to percutaneously introduce an electrophysiology catheter (electrode catheter) having mapping electrodes mounted on its distal extremity. The catheter is maneuvered to place these electrodes in contact with or in close proximity to the endocardium. By monitoring the electrical signals at the endocardium, aberrant conductive tissue sites responsible for the arrhythmia can be pinpointed. Once the origination point for the arrhythmia has been located in the tissue, the physician uses an ablation procedure to destroy the tissue causing the arrhythmia in an attempt to remove the electrical signal irregularities and restore normal heart beat or at least an improved heart beat.

In order to map or otherwise determine the location of a catheter in the heart, location sensing is often performed by a nonfluoroscopic electroanatomical cardiac mapping system. The catheter is equipped with at least one location sensor capable of sensing condition information of the heart chamber and providing three-dimensional position information of the catheter tip in a positional frame of reference. Preferably, the three-dimensional position information is provided by an electromagnetic position sensor that generates signals responsive to the strength of one or more magnetic fields generated by magnetic field radiators external to the patient, the signals being indicative of the three-dimensional position of the sensor in the magnetic fields.

The three-dimensional coordinates of the mapping catheter position sensor are usually determined relative to the position of a reference sensor. The reference sensor is also preferably an electromagnetic sensor that operates according to the same principles as the position sensor in the catheter. The reference sensor may be positioned external to the patient, for example, as part of an adhesive patch applied to the patient's skin. Alternatively, the reference sensor may be positioned internal to the patient, for example, as a component of a reference catheter that is positioned at a particular point in the heart of the patient during the mapping and/or ablation procedure. Thus, the position sensor in the catheter provides the three-dimensional coordinates of the mapping catheter tip in the frame of reference of the position sensor location system relative to the reference position sensor.

However, because the patient is typically awake and breathing and the heart is beating during mapping and ablation procedures, the reference sensor is often moved or shifted relative to the heart. If the reference sensor is internal, blood circulating in the heart or an accidental movement by the operator can dislodge the catheter carrying the reference sensor. If the reference sensor is external, the patient may cough or shift his position. In any case, such a shift in the position of the reference sensor independent of the heart often requires the operator to restart and repeat the mapping procedure. In order to minimize this occurrence, it is desirable that the reference sensor be stabilized against such movement.

A system according to the preamble of claim 1 is known from WO 9806450.

The present invention is directed to a stabilized reference location sensor, particularly one that is carried on a reference catheter. To that end, according to the present invention, there is provided a stabilized catheter system as defined in appended claim 1. In one embodiment, the stabilizing catheter has an elongated catheter body, a distal tip section, and a magnetic member that is situated in the distal tip section, wherein the magnetic member is adapted to attract the reference catheter and stabilize it from moving or shifting within the heart. The stabilizing catheter may be adapted for use in a patient's heart or in a region outside but near the heart, such as the patient's esophagus. The reference and stabilizing catheters can be positioned such that at least one vascular structure of heart extends between them and the magnetic force between them secures the tip section of the reference catheter against one side of the vascular structure. If used in the heart, the stabilizing catheter may carry ring electrodes on its distal tip section.

In another embodiment, the present invention is directed to a catheter stabilizing system, comprising a first catheter having a first magnetic member, a second catheter having a second magnetic member, wherein the magnetic members are attracted to each to stabilize at least one of the catheters against movement while in use in a patient's body. In a more detailed embodiment, the first catheter is adapted for use in the patient's heart and the second catheter is adapted for use outside the patient's heart. Alternatively, the first and the second catheters are adapted for use in the patient's heart. The magnetic member may be carried in the tip section of each catheter such that the respective tip sections are drawn to each other.

Moreover, one of the catheters may be a reference catheter carrying a location sensor, or one of the catheters may be adapted for use in the patient's esophagus. Alternatively, both of the catheters may carry a location sensor and ring electrodes. Nonlimiting examples of suitable embodiments of the magnetic members include a permanent magnet, a ferromagnetic material or an electromagnet, or combinations thereof. Where an electromagnet is used, the current passed therethrough may be DC or AC so that the magnetic members can both attract and repel each other, as desired. The electromagnet embodiment could also be variable in magnetic field strength, so as to aid in fine positioning or adjustment prior to securement.

In use, a vascular structure of the heart may extend between the catheters and the reference catheter is held in contact with the vascular structure.

One of the catheters may be a reference catheter carrying a location sensor. One of the catheters may be adapted for use in the patient's esophagus. At least one of the catheters may be adapted for cardiac ablation.

At least one of the catheters may further comprise an electromagnetic location sensor.

Embodiments of the invention will now be described by way of example with reference to the accompanying drawings, in which:
FIG. 1 is a schematic cross-sectional view of two catheters in use as separated by vascular structures in a patient's body in accordance with the present invention;
FIG. 2 is a schematic perspective view of two catheters in use in a left atrium and an esophagus in a patient's body, respectively, in accordance with the present invention;
FIG. 3 is a schematic perspective view of two catheters in use in a left atrium and a coronary sinus, respectively, in a patient's heart, in accordance with the present invention;
FIG. 4 is a side elevational view of an embodiment of a catheter in accordance with the present invention;
FIG. 5 is a side cross-sectional view of a catheter body and an intermediate section of the catheter of FIG. 4;
FIG. 5a is a longitudinal cross-sectional view of the intermediate section of FIG. 5 taken along lines 5a--5a;
FIG. 6 is a side cross-sectional view of an intermediate section and a tip section of the catheter of FIG. 4;
FIG. 7 is a side cross-sectional view of the control handle of FIG. 4;
FIG. 8 is a side cross sectional view of an alternative embodiment of an intermediate section and a tip section;
FIG. 8a is a longitudinal cross-sectional view of the intermediate section of FIG. 8, taken along line 8a--8a;
FIG. 9 is a side cross sectional view of another alternative embodiment of an intermediate section and a tip section;
FIG. 9a is a longitudinal cross sectional view of the intermediate section of FIG. 9, taken along line 9a--9a;
FIG. 10 is a side cross-sectional view of yet another alternative embodiment of an intermediate section and a tip section;
FIG. 10a is a longitudinal cross sectional view of the intermediate section of FIG. 10, taken along lines 10a-- 10a;
FIG. 11a is a schematic representation of a pair of catheters in accordance with the present invention, both carrying an electromagnet at their respective distal ends;
FIG. 11b is a schematic representation of a pair of catheters in accordance with the present invention, one carrying an electromagnet and the other carrying a permanent magnet at their respective distal ends;
FIG. 11c is a schematic representation of a pair of catheters in accordance with the present invention, one carrying an electromagnet and the other carrying a ferromagnetic material at their respective distal ends;
FIG. 11d is a schematic representation of a pair of catheters in accordance with the present invention, both carrying a permanent magnet at their respective distal ends;
FIG. 11e is a schematic representation of a pair of catheters in accordance with the present invention, one carrying a permanent magnet and the other carrying a ferromagnetic material at their respective distal ends;
FIG. 12 is a side cross sectional view of yet another alternative embodiment of an intermediate section and a tip section in accordance with the present invention; and
FIG. 13 is a side cross-sectional view of yet another alternative embodiment of an intermediate section and a tip section in accordance with the present invention.

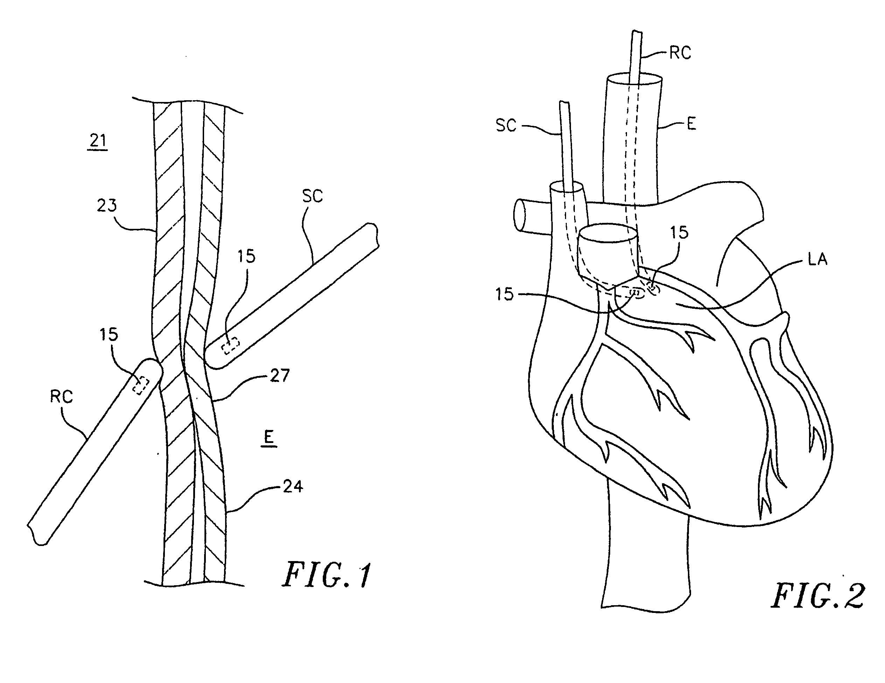

Referring to Fig. 1, the present invention provides a stabilizing catheter SC for securing a reference catheter RC against shifting or moving while in use in a heart chamber 21. In accordance with a feature of the present invention, the stabilizing catheter SC and the reference catheter are each equipped with a magnetic member 15 at or near their distal tips so as to be drawn toward each other by a magnetic force in stabilizing the reference catheter against a vascular structure 23 extending between the two catheters. With reference to FIG. 1 and an application illustrated in FIG. 2, the reference catheter RC is positioned in the left atrium LA and the stabilizing catheter SC is positioned in the patient's esophagus E. The magnetic force between the magnetic members 15 passes through the vascular structure 23 of the heart and the vascular structure of the esophagus 24 in drawing the tip section of the reference catheter toward a posterior wall 23 of the left atrium and the tip section of the stabilizing catheter toward an anterior wall 24 of the esophagus to hold the reference catheter RC in place against the posterior wall 23.

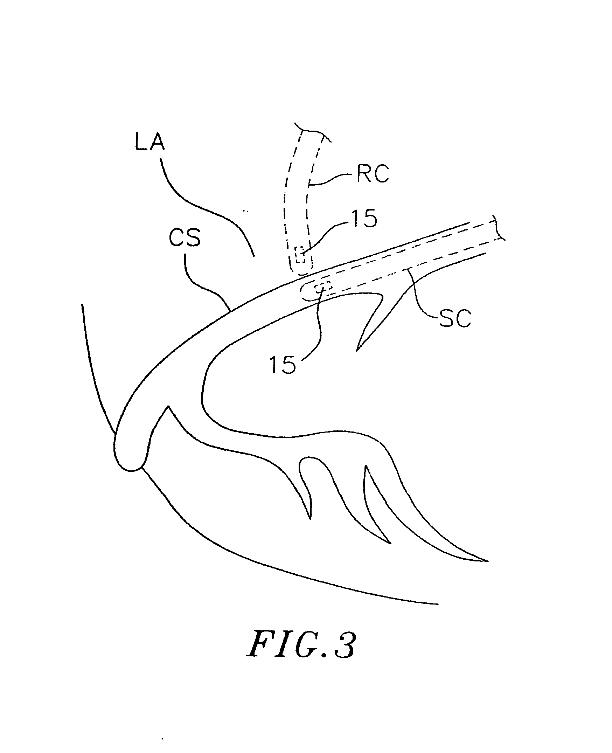

In the application illustrated in FIG. 3, the reference catheter RC is positioned in the left atrium LA while the stabilizing catheter SC is positioned in the coronary sinus CS, a generally more confining region of the heart. The magnetic force between the two catheters passes through the vascular structure of the heart in drawing the tip section of the reference catheter toward the posterior wall of the left atrium and the stabilizing catheter toward an anterior wall of the coronary sinus to hold the reference catheter in place against the posterior wall of the left atrium.

It is understood by one of ordinary skill in the art that the two catheters be placed in any other locations within or near the heart to carry out the same function of the stabilizing catheter holding the reference catheter generally stationary at a location of choice. Moreover, the two catheters may be separated by only a single vascular structure, or by two or more vascular structures.

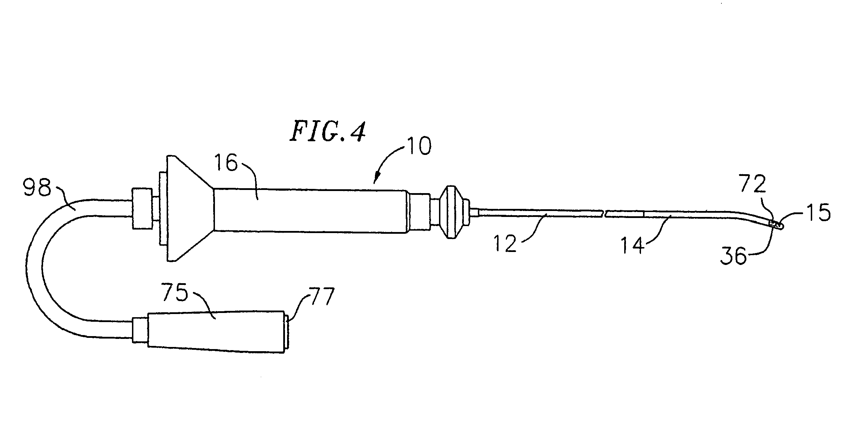

As shown in FIG. 4, the reference catheter RC comprises an elongated catheter body 12 having proximal and distal ends, a deflectable intermediate section 14 at the distal end of the catheter body, a control handle 16 at the proximal end of the catheter body, and a tip section 36 mounted at the distal end of the intermediate section. The tip section 36 carries an electromagnetic sensor 72 for location sensing of the tip section and a magnetic member 15 adapted for magnetic attraction with the stabilizing catheter SC.

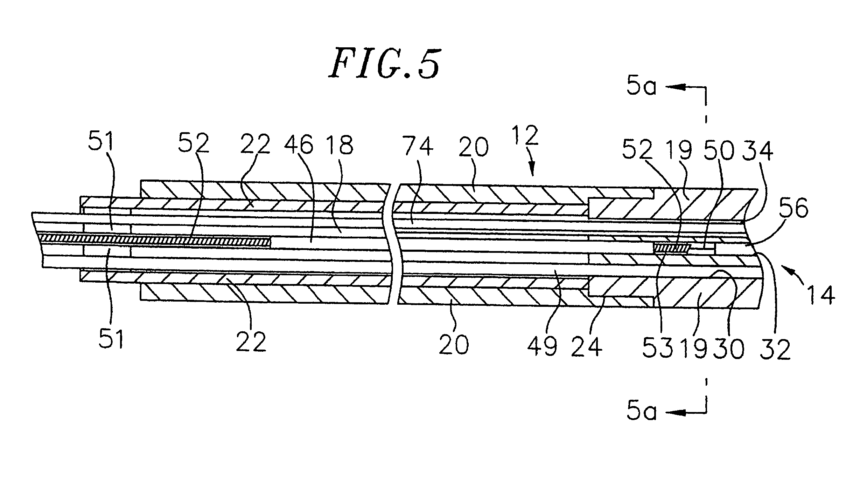

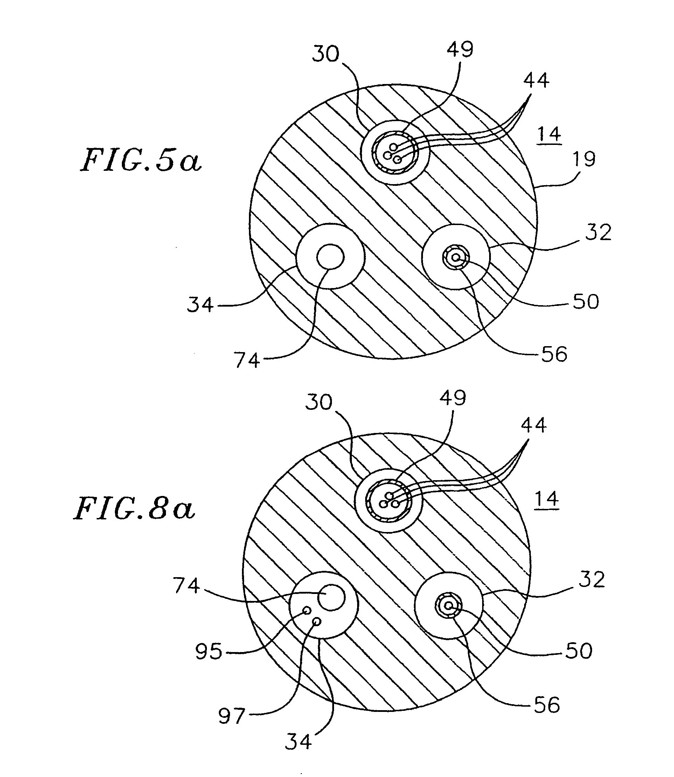

With reference to FIG. 5, the catheter body 12 comprises an elongated tubular construction having a single, axial or central lumen 18. The catheter body 12 is flexible, i.e., bendable, but substantially non-compressible along its length. The catheter body 12 can be of any suitable construction and made of any suitable material. A presently preferred construction comprises an outer wall 20 made of polyurethane or PEBAX. The outer wall 20 comprises an imbedded braided mesh of stainless steel or the like to increase torsional stiffness of the catheter body 12 so that, when the control handle 16 is rotated, the intermediate section 14 of the catheter 10 will rotate in a corresponding manner.

The outer diameter of the catheter body 12 is not critical, but is preferably no more than about 8 french, more preferably 7 french. Likewise the thickness of the outer wall 20 is not critical, but is thin enough so that the central lumen 18 can accommodate a puller wire, lead wires, and any other desired wires, cables or tubes. If desired, the inner surface of the outer wall 20 is lined with a stiffening tube 22 to provide improved torsional stability. A particularly preferred catheter has an outer wall 20 with an outer diameter of from about 2.29 mm (0.090 inch) to about 2.39 mm (0.94 inch) and an inner diameter of from about 1.55 mm (0.061 inch) to about 1.65 mm (0.065 inch).

As shown in FIGs. 5 and 5a, the intermediate section 14 distal the catheter body 12 comprises a short section of tubing 19 having multiple lumens. A first lumen 30 carries electrode lead wires 44. A second lumen 32 carries a puller wire 50. A third lumen 34 carries a cable 74 for the electromagnetic location sensor 72. The tubing 19 is made of a suitable nontoxic material that is preferably more flexible than the catheter body 12. A presently preferred material for the tubing 19 is braided polyurethane, i.e., polyurethane with an embedded mesh of braided stainless steel or the like. The size of each lumen is not critical, but is sufficient to house the lead wires, puller wire, the electromagnetic sensor cable or any other components.

The useful length of the catheter, i.e., that portion that can be inserted into the body can vary as desired. Preferably the useful length ranges from about 110 cm to about 125 cm. The length of the intermediate section 14 is a relatively small portion of the useful length, and preferably ranges from about 3.5 cm to about 10 cm, more preferably 6 from about 5 cm to about 6.5 cm.

A preferred means for attaching the catheter body 12 to the intermediate section 14 is illustrated in FIG. 5. The proximal end of the intermediate section 14 comprises an outer circumferential notch 24 that receives the inner surface of the outer wall 20 of the catheter body 12. The intermediate section 14 and catheter body 12 are attached by glue or the like.

If desired, a spacer (not shown) can be located within the catheter body between the distal end of the stiffening tube (if provided) and the proximal end of the intermediate section. The spacer provides a transition in flexibility at the junction of the catheter body and intermediate section, which allows this junction to bend smoothly without folding or kinking. A catheter having such a spacer is described in US-5,964,757.

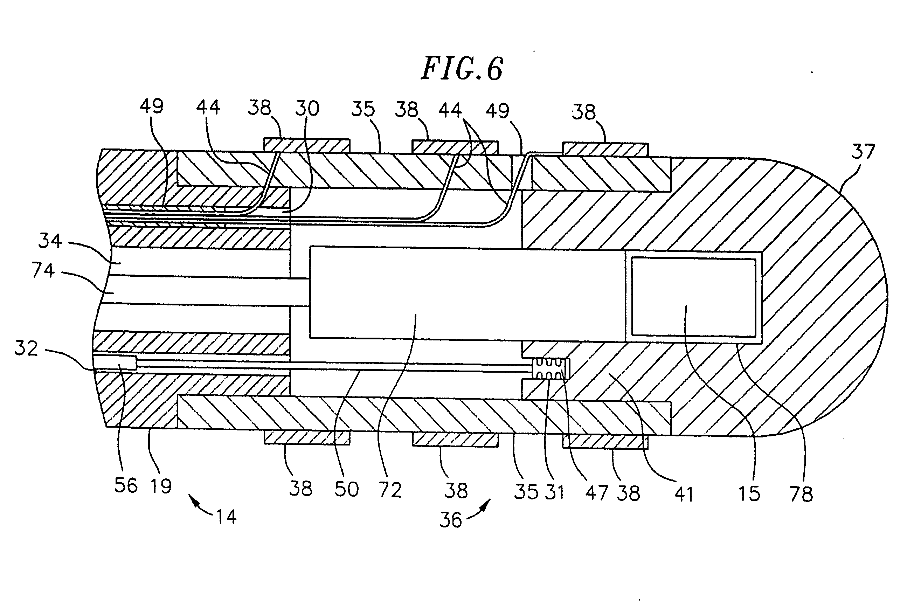

Referring to FIG. 6, at the distal end of the intermediate section 14 is a tip section 36. Preferably the tip section 36 has a diameter about the same as the outer diameter of the tubing 19. As illustrated in the embodiment of FIG. 6, the tip section 14 has a tip dome 37 and a plastic housing or a short section of tubing 35 proximal the tip dome 37. The proximal end of the plastic housing 35 is received by an outer circumferential notch formed in the distal end of the tubing 19 and is bonded thereto with polyurethane glue or the like. Preferably the plastic housing is about 1 cm long.

The tip dome 37 is generally solid, having a blind hole 31 that generally corresponds in size and location to the second lumen 32 carrying the puller wire 50, and a cavity 39 (e.g., a bore) that generally corresponds in location to the third lumen 34 carrying the sensor cable 74. The blind hole 31 and the cavity 39 extend from the proximal end of the tip dome 37, but do not extend through to the distal end of the tip dome. A preferred tip dome has an effective length, i.e., from its distal end to the distal end of the tubing 35, of about 3.5 mm, and an actual length, i.e., from its distal end to its proximal end, of about 4.0 mm.

The tip dome 37 is attached to the plastic housing 35 by a stem 41 extending from the proximal end of the tip dome 37, which is received by the distal end of the tubing 35. The stem is affixed with adhesive, glue or the like. The puller wire 50, the lead wires 44, the sensor cable 74 that extend into the dome tip 36 help keep the tip dome in place on the tip section.

In the embodiment shown in FIG. 6, there are three ring electrodes 38 mounted on the tubing 35 of the tip section 36. The tip dome 37 and ring electrodes 38 can be made of any suitable material, for example, from machined platinum-iridium bar (90% platinum/10% iridium).

The ring electrodes 38 are each connected to a separate lead wire 44. The lead wires 44 extend through the first lumen 30 of intermediate section 14, the central lumen 18 of the catheter body 12, and the control handle 16, and terminate at their proximal end in an input jack (not shown) that may be plugged into an appropriate monitor (not shown). The portion of the lead wires 44 extending through the central lumen 18 of the catheter body 12, control handle 16 and proximal end of the intermediate section 14 are enclosed within a protective, nonconducting sheath 49, which can be made of any suitable material, preferably polyimide. The sheath 49 is anchored at its distal end to the distal end of the intermediate section 14 by gluing it in the first lumen 30 with polyurethane glue or the like.

The lead wires 44 are attached to the ring electrodes 38 by any conventional technique. Connection of a lead wire 44 to a ring electrode 38 is preferably accomplished by first making a small hole through the tubing 35. Such a hole can be created, for example, by inserting a needle through the tubing and heating the needle sufficiently to form a permanent hole. A lead wire is then drawn through the hole by using a microhook or the like. The ends of the lead wire are then stripped of any coating and soldered or welded to the underside of the ring electrode 38, which is then slid into position over the hole and fixed in place with polyurethane glue or the like.

Due to the length of the plastic housing 35, the most distal ring electrode 38 is mounted on the plastic housing 21 at a position above the stem 41 of the tip dome 37. As a result, the lead wire 44 for the most distal ring electrode 38 extends though a hole 49 in the plastic housing 35 that is proximal to the distal ring electrode 38 and stem 41. The lead wire 44 extends a short distance along the outside of the plastic housing 35 and is soldered to the underside of the most distal ring electrode 38. Polyurethane glue or the like is used to cover the exposed section of the lead wire 44 and to fill in the hole 49.

The ring electrodes 38 allow an operator to collect electrophysiological data from the tip section 36 of the reference catheter RC. Accordingly, the presence and number of ring electrodes 38 can vary as desired. Alternatively, one or more proximal ring electrodes 38 can be positioned over the flexible tubing 19 of the intermediate section 14.

In accordance with a feature of the present invention, the tip section 36 carries the magnetic member 15 for magnetic attraction with the stabilizing catheter SC. In the embodiment of FIG. 6, the magnetic member 15 is situated in the distal end of the cavity 39 in the tip dome 37. The magnetic member 15 may be made of any suitable material or be of any configuration as described below in further detail. In that regard, it is understood by one of ordinary skill in the art that the tip dome 37 in its entirety can be constructed of a magnetic material, if appropriate or desired.

In the illustrated embodiment of Fig. 6, the distal end of the electromagnetic location sensor 72 is proximal the magnetic member 15 and in an abutting relationship therewith in the cavity 39. The distal end of the location sensor 72 is also fixedly bonded in the cavity by adhesive, glue or the like. The proximal end of the location sensor 72 extends proximally in the plastic housing 35 and is generally aligned with the third lumen 34 of the tubing 19 through which the sensor cable 74 extends from the proximal end of the location sensor 72.

The electromagnetic sensor cable 74 extends through the third lumen 34 of the tip section 14, through the central lumen 18 of the catheter body 12, and into the control handle 16. As shown in FIG. 4**,** the electromagnetic sensor cable 74 then extends out the proximal end of the control handle 16 within an umbilical cord 98 to a sensor control module 75 that houses a circuit board (not shown). Alternatively, the circuit board can be housed within the control handle 16, for example, as described in US-5964757. The electromagnetic sensor cable 74 comprises multiple wires encased within a plastic covered sheath. In the sensor control module 75, the wires of the electromagnetic sensor cable 74 are connected to the circuit board. The circuit board amplifies the signal received from the electromagnetic sensor 72 and transmits it to a computer in a form understandable by the computer by means of the sensor connector 77 at the proximal end of the sensor control module 75, as shown in FIG. 4. Also, because the catheter is designed for single use on!y, the circuit board may contain an EPROM chip which shuts down the circuit board approximately 24 hours after the catheter has been used. This prevents the catheter, or at least the electromagnetic sensor, from being used twice. Suitable electromagnetic sensors for use with the present invention are described, for example, in US-5,558,091, US-5,443,489, US-5,480,422, US-5,546,951, US-5,568,809, and US-5,391,199 and International Publication No. WO-A-95/02995. A preferred electromagnetic mapping sensor 72 has a length of from about 6 mm to about 7 mm and a diameter of about 1.3 mm.

Referring back to FIG. 5, the puller wire 50 extends through the catheter body 12, is anchored at its proximal end to the control handle 16, and is anchored at its distal end to the tip dome 37. The puller wire is made of any suitable metal, such as stainless steel or Nitinol, and preferably has a coating of Teflon.RTM. or the like. The coating imparts lubricity to the puller wire. The puller wire preferably has a diameter ranging from about 0.15 mm (0.006 inch) to about 0.25 mm (0.010 inch).

A compression coil 52 is situated within the catheter body 12 in surrounding relation to the puller wire 50. The compression coil 52 extends from the proximal end of the catheter body 12 to the proximal end of the intermediate section 14. The compression coil 52 is made of any suitable metal, preferably stainless steel. The compression coil 52 is tightly wound on itself to provide flexibility, i.e., bending, but to resist compression. The inner diameter of the compression coil is preferably slightly larger than the diameter of the puller wire 50. The Teflon coating on the puller wire 50 allows it to slide freely within the compression coil 52. If desired, particularly if the lead wires 50 are not enclosed by a protective sheath 49, the outer surface 26 of the compression coil can be covered by a flexible, non-conductive sheath, e.g., made of polyimide tubing, to prevent contact between the compression coil 52 and any other wires within the catheter body 12.

The compression coil 52 is anchored at its proximal end to the proximal end of the stiffening tube 22 in the catheter body 12 by glue joint 53 and at its distal end to the tip section 14 by glue joint 51. Both glue joints 53 and 51 preferably comprise polyurethane glue or the like. The glue may be applied by means of a syringe or the like through a hole made between the outer surface of the catheter body 12 and the central lumen 18. Such a hole may be formed, for example, by a needle or the like that punctures the outer wall 20 of the catheter body 12 and the stiffening tube 22 which is heated sufficiently to form a permanent hole. The glue is then introduced through the hole to the outer surface of the compression coil 52 and wicks around the outer circumference to form a glue joint about the entire circumference of the compression coil 52.

As shown in FIG. 6, the puller wire 50 is anchored at its distal end to the tip dome 37 within the blind hole 31. A preferred method for anchoring the puller wire 42 within the tip electrode 36 is by crimping metal tubing 47 to the distal end of the puller wire 50 and soldering the metal tubing 47 inside the blind hole 31. Anchoring the puller wire 50 within the tip dome 37 provides additional support, reducing the likelihood that the tip dome 37 will fall off the tip section 36. Alternatively, the puller wire 50 can be attached to the tubing 35 of the tip section 36, or the distal section of the tubing 19 of the intermediate section 14. Within the second lumen 32 of the intermediate section 14, the puller wire 50 extends through a plastic, preferably Teflon, sheath 56, which prevents the puller wire 42 from cutting into the wall of the tubing 19 when the intermediate section 14 is deflected.

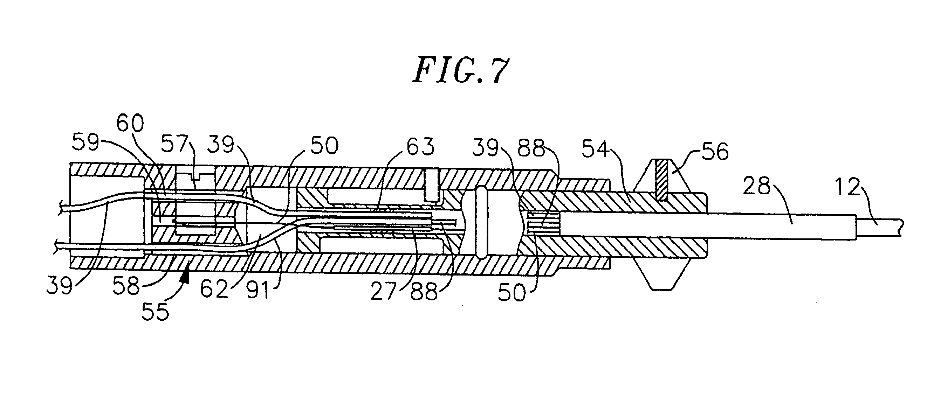

Longitudinal movement of the puller wire 50 relative to the catheter body 12, which results in deflection of the intermediate section 14, is accomplished by suitable manipulation of the control handle 16. As shown in FIG. 7, the distal end of the control handle 16 comprises a piston 54 with a thumb control 56 for manipulating the puller wire 50. The proximal end of the catheter body 12 is connected to the piston 54 by means of a shrink sleeve 28.

The puller wire 50, lead wires 44, the sensor cable 74 extend through the piston 54. The puller wire 50 is anchored to an anchor pin 57, located proximal to the piston 54. Within the piston 54, the sensor cable 74 extends into another protective sheath 91, preferably made of polyurethane. The protective sheathes 49 and 91 are anchored to the piston 54, preferably by polyurethane glue or the like at a glue joint 53, allowing the lead wires 50 and the sensor cable 74 longitudinal movement within the control handle 16 so that they do not break when the piston 54 is adjusted to manipulate the puller wire 50. Within the piston 54, the puller wire 50 extends through a transfer tube 27, preferably a polyimide tube, to allow longitudinal movement of the puller wire near the glue joint 63.

The mechanics and operation of the control handle are described in US-6602242. It is understood by one of ordinary skill in the art that other control handles for manipulating the puller wire or puller wires (for bi-directional deflection) may be used with the present catheters.

The stabilizing catheter SC for use in conjunction with the reference catheter RC may have a construction similar to that of the reference catheter of FIGs. 4, 5 and 5a, for example, in terms of having an elongated catheter body 12 with proximal and distal ends, a intermediate section 14 at the distal end of the catheter body, a control handle 16 at the proximal end of the catheter body, and a tip section 36 mounted at the distal end of the intermediate section, where the tip section 36 carries an electromagnetic sensor 72 for location sensing of the tip section and a magnetic member 15 for magnetic attraction. Accordingly, the embodiment of the tip section 36 shown in FIG. 6 is also suitable as a tip section 36 for the stabilizing catheter. In that regard, except as noted herein, similar structures shared by the catheters RC and SC are identified by similar reference numerals.

In accordance with the present invention, the reference and the stabilizing catheters are attracted to each other through magnetic force between the respective magnetic members. In that regard, each magnetic member can be a source of a magnetic field, can interact with another magnetic field or can influence a material to exhibit magnetic behaviors. Therefore, suitable nonlimiting examples of the magnetic member 15 include electromagnets, permanent magnets and ferromagnets. Electromagnets may be current-carrying coils and solenoids, with or without a metal core. Permanent magnets are materials where magnetic fields of individual atoms are aligned in one direction, giving rise to a net magnetic field. Ferromagnets are materials with domains in which the magnetic fields of individual atoms align, but the orientation of the magnetic fields of the domains is random, giving rise to no net magnetic field. When an external magnetic field is applied to them, the magnetic fields of the individual domains tend to line up in the direction of this external field, due to the nature of the magnetic forces, which causes the external magnetic field to be enhanced.

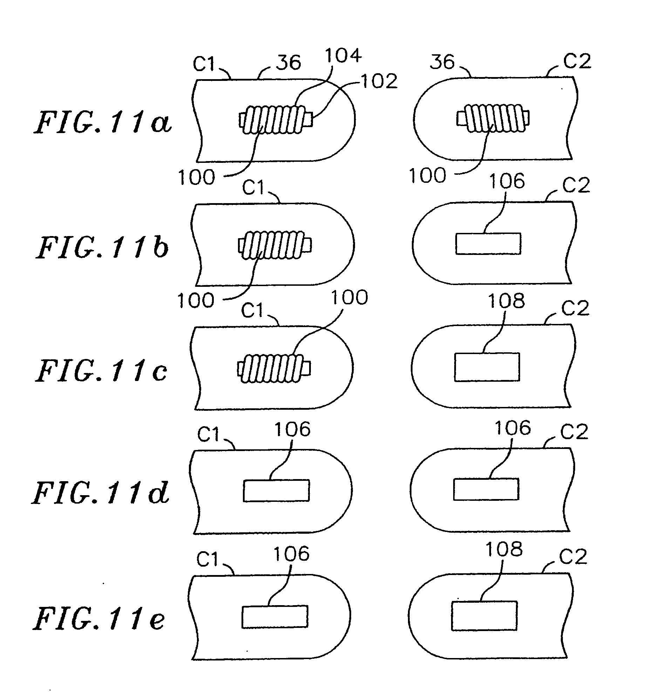

In view of the foregoing, the present invention contemplates different combinations of suitable examples of the magnetic member carried in the tip sections of a first and a second catheter C1 and C2 as illustrated in FIGs. 11a-11e. In particular, FIG. 11a illustrates the magnetic member 15 of each of the catheters C1 and C2 as an electromagnet 100 with a metal core 102 and a surrounding coil 104. Fig. 1b illustrates the magnetic member 15 of the second catheter C2 as a permanent magnet 106(Fig. 11b). FIG. 11c illustrates the magnetic member 15 of the second catheter C2 as a ferromagnetic material 108 (Fig. 11c).

Alternatively, Fig. 11d illustrates the magnetic members 15 of both catheters C1 and C2 as permanent magnets 106. And, Fig. 11e illustrates the magnetic member 15 of the second catheter C2 is of a ferromagnetic material 108.

Where the magnetic member 15 is a permanent magnet 106 or a ferromagnetic material 108, the tip section 36 and the intermediate section 14 of either a reference catheter or a stabilizing catheter may adopt the embodiments of FIGs 6 and 6a.

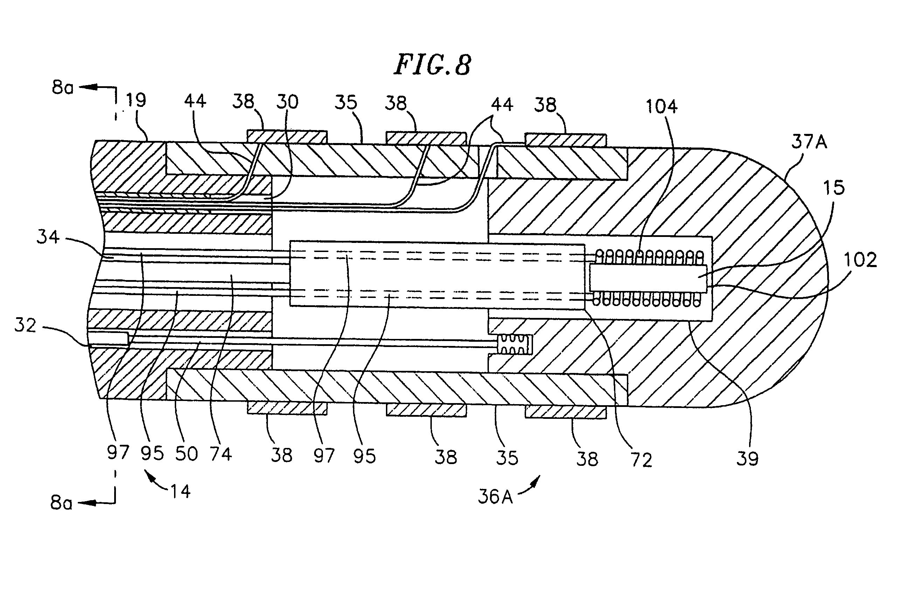

Where the magnetic member 15 is an electromagnet, the tip section 36 and the intermediate section 14 of either a reference catheter or a stabilizing catheter may adopt the embodiments of FIGs. 8 and 8a.

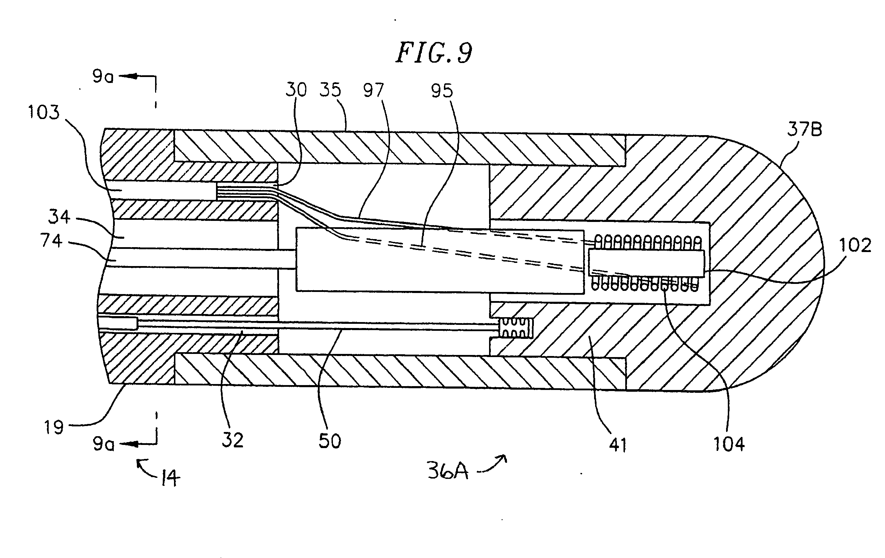

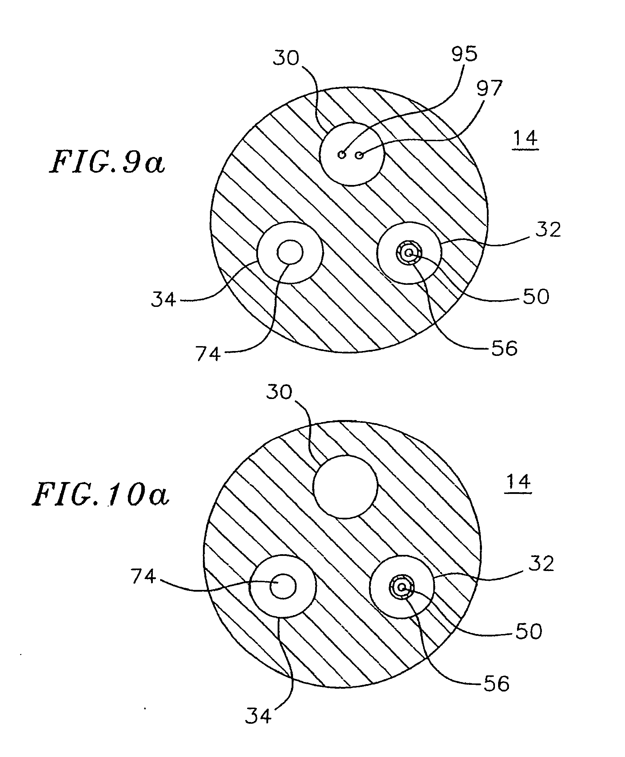

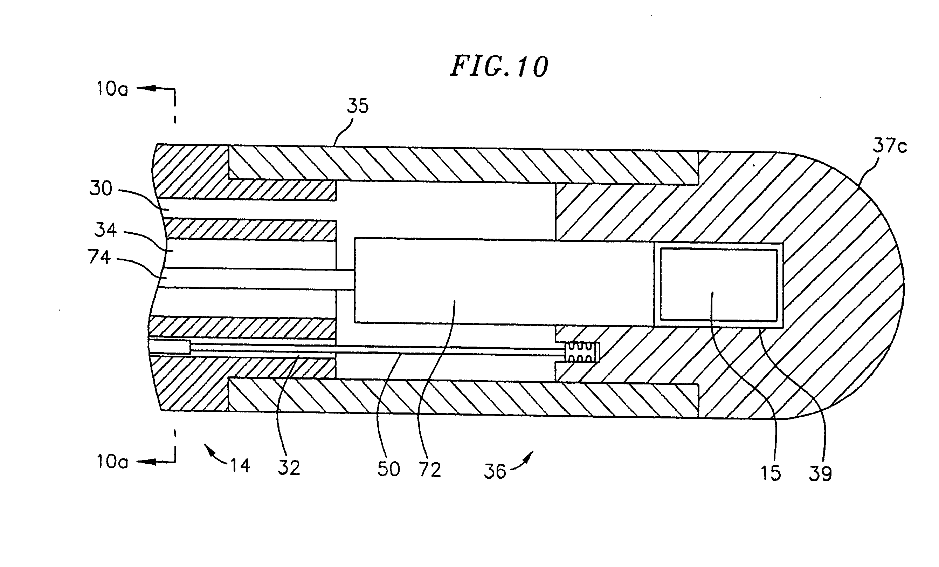

However, where the stabilizing catheter SC is without ring electrodes, particularly where the stabilizing catheter is positioned outside the heart, e.g., in the patient's esophagus, the tip section 36 and the intermediate section 14 may adopt the embodiments of FIGs. 9 and 9a (where the magnetic member is an electromagnet 100) or FIGs. 10 and 10a, (where the magnetic member is a permanent magnet 106 or a ferromagnetic material 108).

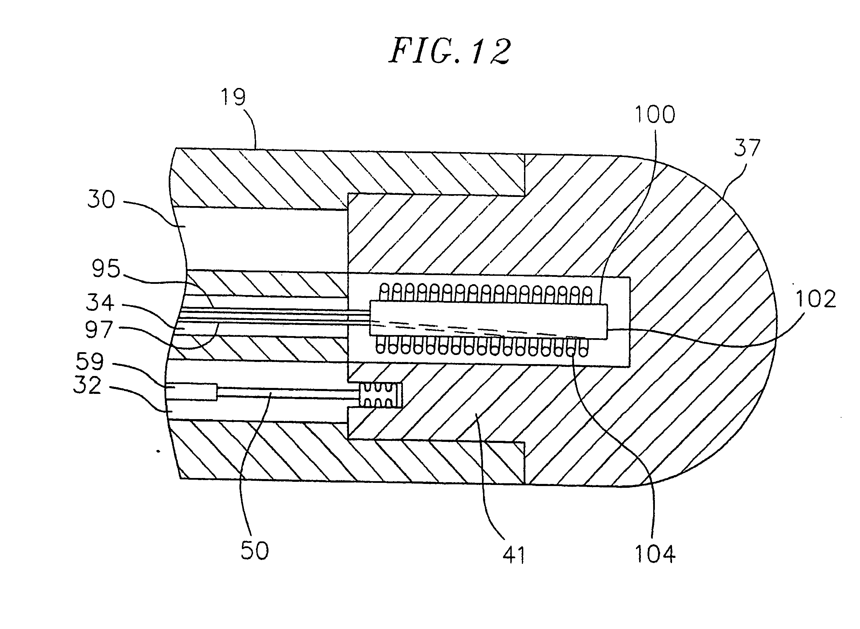

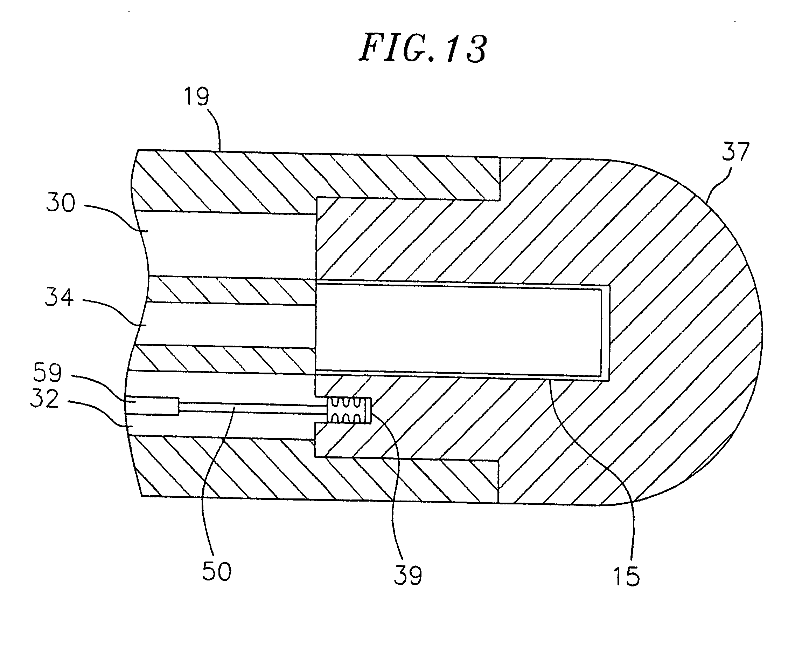

Moreover, FIGs. 12 and 13 illustrate additional embodiments of the stabilizing catheter SC suitable for use outside the heart, as the stabilizing catheter SC carries no ring electrodes and no location sensor. In these embodiments, the tip dome 37 is attached to the tubing 19 of the intermediate section by inserting the stem 41 into an inner circumferential notch in the distal end of the tubing 19 and bonded thereto by adhesive, glue or the like. In the embodiment of FIG. 12, the magnetic member 15 is an electromagnet. In the embodiment of FIG. 13, the magnetic member 15 is a permanent magnet or a ferromagnetic material.

It is understood by one of ordinary skill in the art that a catheter may contain a plurality of the foregoing suitable examples of the magnetic member, or different combinations of such examples, as desired or appropriate.

In the embodiments of FIGs. 8 and 9 and 12, where the magnetic member 15 is an electromagnet 100, the coil 104 has a feed wire 97 and a return wire 99 for passing a current through the coil 104. In the embodiment of FIG. 8, the coil wires 95 and 97 extend through the third lumen 34 along with the sensor cable 74. In the embodiment of FIG. 9, the coil wires 95 and 97 extend through the first lumen 30. In the embodiment of FIG. 12, the coil wires 95 and 97 extend through the third lumen 34 of the tubing 19. In any case, the coil wires extend through the central lumen 18 of the catheter body 12 and through the sheath 91 in the control handle. The coil wires then extend out the proximal end of the control handle 16 (separately from the sensor cable 74) to a power supply (not shown). The coil wires may pass through nonconducting sheath(s), including e.g., sheath 103, as appropriate between their proximal and distal ends in the control handle and the tip section 36A.

The current passing through the coil to generate the magnetic field may be DC or AC, as appropriate. In fact, the current may be reversed as suitable or appropriate to repel the reference catheter RC. In addition, the current may be variable, allowing the magnetic field to be gradually strengthened as the catheter nears its final position. The current could then be increased to a maximum securing strength once the final position is achieved. As noted, a variable field could provide fine adjustment. It is understood by one of ordinary skill in the art that the magnitude or strength of the magnetic field is sufficient to pass through vascular structures of the heart or other tissue situated between the two catheters, and to stabilize the reference catheter against most movement caused by circulating blood, the beating heart and/or shifting of the patient's body.

It is understood by one of ordinary skill in the art that position or orientation of the magnetic member in the tip section may differ, provided the position and/or orientation facilitates the stabilization of the distal tip of reference catheter or the portion thereof that carries the location sensor. To that end, it may be preferred to situate the magnetic member closer to than farther from the location sensor, wherever the location sensor may be situated in the reference catheter. Moreover, any of the tip domes described herein may be a tip electrode in that an additional lead wire can be welded to a second blind hole in the proximal end of the tip dome. The present invention also contemplates providing magnetic means in an external reference patch that is placed in a fixed position on the patient's back.

Under fluoroscopic guidance, or other suitable guidance means, the reference catheter RC and the stabilizing catheter SC are introduced into the patient's body. The reference catheter RC is advanced into the patient's heart through appropriate vascular access and positioned inside the heart chamber. The stabilizing catheter may be positioned also in the heart, e.g., in the coronary sinus, or outside the heart, e.g., the patient's esophagus. In any case, the catheters RC and SC are preferably positioned on opposite sides of at least one layer of vascular structure. Where the stabilizing catheter is also positioned in the heart, the ring electrodes may be used to detect electrical activity in the heart muscle.

To secure the reference catheter from moving from its position in the heart as a means to stabilize its location sensor that is carried in the tip section, the tip section of the stabilizing catheter is maneuvered into close proximity to the tip section of the reference catheter. Where the magnetic member of either or both of the catheters is an electromagnet, a current is passed through the electromagnet via coil wires 95 and 97 to generate an attractive magnetic force between the magnetic members. In any case, when the magnetic member are sufficiently close to each other, the magnetic force draws the tip sections together with sufficient force that each catheter tip section contacts the vascular structure(s) extending between them which serve as a supporting structure to stabilize and hold the reference catheter against movement caused by the heart beating, circulating blood, movement of the patient and/or inadvertent disturbance by the operator.

When the reference catheter is to be repositioned or no longer needs to be held in position against the vascular structure, one or both of the catheters can be maneuvered by advancement, withdrawal or deflection of the catheters to separate the tip sections. In the instance where the magnetic member of at least one of the catheters is an electromagnet and driven by an AC current, the current can be reversed to reverse the magnetic field and repel the magnetic member of the other catheter.

## Claims

1. A catheter stabilizing system comprising:
a stabilizing element (SC) comprising:
a body (12);
a distal tip section (36); and
a first magnetic member (15) in the distal tip section (36); and a cardiac reference catheter (RC) comprising a second magnetic member (5);
wherein the first magnetic member (15) is adapted to attract the second magnetic member (15) of the reference catheter (RC) in use thereby stabilising at least one of the catheters against movement while in use in a patient's body, **characterized in that** said stabilizing element is a catheter.

2. A catheter stabilizing system of claim 1, wherein the first and/or second magnetic member (15) is a permanent magnet (106).

3. A catheter stabilizing system of claim 1, wherein the first and/or second magnetic member (15) is of a ferromagnetic material (108).

4. A catheter stabilizing system of claim 1, wherein the first and/or second magnetic member (15) is an electromagnet (100).

5. A catheter stabilizing system of claim 1, wherein the stabilizing catheter (SC) is adapted for use in a patient's heart.

6. A catheter stabilizing system of claim 1, wherein the stabilizing catheter (SC) is adapted for use in a patient's esophagus.

7. A catheter stabilizing system of claim 1, wherein the stabilizing catheter (SC) is adapted for use in a region near the heart.

8. A catheter stabilizing system of claim 1, wherein the stabilizing catheter (SC) further comprises ring electrodes (38) on the distal tip section (36).

9. A catheter stabilizing system of claim 1, wherein the reference catheter (RC) comprises an electromagnetic location sensor (72).

## Patentansprüche

1. Einen Katheter stabilisierendes System, das aufweist:
ein stabilisierendes Element (SC), mit:
einem Körper (12);
einem distalen Spitzenabschnitt (36); und
einem ersten magnetischen Element (15) in dem distalen Spitzenabschnitt (36); und
einen Herz-Referenzkatheter (RC), der ein zweites magnetisches Element (5) aufweist;
wobei das erste magnetische Element (15) dazu ausgelegt ist, das zweite magnetische Element (15) des Referenzkatheters (RC) in der Verwendung anzuziehen, so dass wenigstens einer der Katheter gegen Bewegung stabilisiert wird, während sie im Körper eines Patienten im Einsatz sind, **dadurch gekennzeichnet, dass** das stabilisierende Element ein Katheter ist.

2. Einen Katheter stabilisierendes System nach Anspruch 1, bei dem das erste und/oder das zweite magnetische Element (15) ein Dauermagnet (106) ist.

3. Einen Katheter stabilisierendes System nach Anspruch 1, bei dem das erste und/oder das zweite magnetische Element (15) aus einem ferromagnetischen Material (108) besteht.

4. Einen Katheter stabilisierendes System nach Anspruch 1, bei dem das erste und/oder das zweite magnetische Element (15) ein Elektromagnet (100) ist.

5. Einen Katheter stabilisierendes System nach Anspruch 1, bei dem der stabilisierende Katheter (SC) für den Einsatz im Herzen eines Patienten ausgelegt ist.

6. Einen Katheter stabilisierendes System nach Anspruch 1, bei dem der stabilisierende Katheter (SC) für den Einsatz in der Speiseröhre eines Patienten ausgelegt ist.

7. Einen Katheter stabilisierendes System nach Anspruch 1, bei dem der stabilisierende Katheter (SC) zum Einsatz in einem Bereich nahe dem Herzen ausgelegt ist.

8. Einen Katheter stabilisierendes System nach Anspruch 1, bei dem der stabilisierende Katheter (SC) weiter Ringelektroden (18) auf dem distalen Spitzenabschnitt (36) aufweist.

9. Einen Katheter stabilisierendes System nach Anspruch 1, bei dem der Referenzkatheter (RC) einen elektromagnetischen Ortssensor (72) aufweist.

## Revendications

1. Système de stabilisation de cathéter comprenant :
✔ un élément de stabilisation (SC) comprenant :
✔ un corps (12) ;
✔ une partie d'extrémité distale (36) ; et
✔ un premier élément magnétique (15) dans la partie d'extrémité distale (36) ; et
✔ un cathéter de référence cardiaque (RC) comprenant un deuxième élément magnétique (5) ;
dans lequel le premier élément magnétique (15) est adapté pour attirer le deuxième élément magnétique (15) du cathéter de référence (RC) stabilisant ainsi lors de l'utilisation au moins l'un des cathéters contre un mouvement lors de l'utilisation dans le corps d'un patient, **caractérisé en ce que** ledit élément de stabilisation est un cathéter.

2. Système de stabilisation de cathéter selon la revendication 1, dans lequel le premier et/ou le deuxième élément magnétique (15) est un aimant permanent (106).

3. Système de stabilisation de cathéter selon la revendication 1, **caractérisé en ce que** le premier et/ou le deuxième élément magnétique (15) est en matériau ferromagnétique (108).

4. Système de stabilisation de cathéter selon la revendication 1, dans lequel le premier et/ou le deuxième élément magnétique (15) est un électroaimant (100).

5. Système de stabilisation de cathéter selon la revendication 1, dans lequel le cathéter de stabilisation (SC) est adapté pour l'utilisation dans le coeur d'un patient.

6. Système de stabilisation de cathéter selon la revendication 1, dans lequel le cathéter de stabilisation (SC) est adapté pour une utilisation dans l'oesophage d'un patient.

7. Système de stabilisation de cathéter selon la revendication 1, dans lequel le cathéter de stabilisation (SC) est adapté pour l'utilisation dans une région proche du coeur.

8. Système de stabilisation de cathéter selon la revendication 1, dans lequel le cathéter de stabilisation (SC) comprend en outre des électrodes annulaires (38) sur la partie d'extrémité distale (36).

9. Système de stabilisation de cathéter selon la revendication 1, dans lequel le cathéter de référence (RC) comprend un détecteur d'emplacement électromagnétique (72).
